# EUROPEAN PATENT SPECIFICATION

(11) **EP 4 370 176 B1**
(45) Date of publication and mention of the grant of the patent: **13.11.2024**
(21) Application number: 22762208.1
(22) Date of filing: 10.08.2022
(51) Int. Cl.: A61M 5/28, A61J 1/06, A61M 5/32

(54) **PRE-FILLED MEDICAL DELIVERY ASSEMBLY WITH A NEEDLE SHIELD**
VORGEFÜLLTE MEDIZINISCHE ABGABEANORDNUNG MIT NADELSCHUTZ
ENSEMBLE DE DISTRIBUTION MÉDICALE PRÉ-REMPLI AVEC PROTECTION D'AIGUILLE

(30) Priority: 10.08.2021 US 202163231688 P
(43) Date of publication of application: 22.05.2024
(73) Proprietor: Koska Family Limited, Eastbourne Sussex BN21 4PT (GB)
(72) Inventor: CARTER, Chelsea, Durham, NC 27713 (US); CHA, Jae-Hyok, Gongju-si Chungcheongnam-do TN223RN (KR); KOSKA, Marc, Sussex BN21 4PT (GB); IN, Hanjin, Toronto, Ontario M5S0A6 (CA); PRICE, Jeff, Windermere, FL 34786 (US)
(74) Representative: Mewburn Ellis LLP
(86) International application number: PCT/US2022/040006
(87) International publication number: WO 2023/018840

(56) References cited:
- US-A1- 2005 049 560
- US-A1- 2009 171 311
- US-A1- 2015 283 332
- US-A1- 2021 128 835

## Description

### CROSS-REFERENCE TO RELATED APPLICATIONS

This application claims benefit and priority under 35 U.S.C. §119(e) to, and is a Non-provisional of, U.S. Provisional Patent Application No. 63/231,688 filed on August 10, 2021 and titled "SYSTEMS AND METHODS FOR PRE-FILLED MEDICAL DELIVERY ASSEMBLIES".

### BACKGROUND

Applicant has previously developed various single-use, single-dose, pre-filled and/or disposable medical injection devices and systems, such as ones utilizing a fluid container manufactured in accordance with a Blow-Fill-Seal (BFS) manufacturing process and mated to one or more components comprising an administration member for injecting a fluid (*e.g.,* a pharmacological agent) from the BFS container into a patient. Applicant's U.S. Patent Application US 2022/0323301 A1 titled SYSTEMS AND METHODS FOR FLUID DELIVERY is one example of some embodiments of such injection devices and systems. Such injection devices and systems may benefit from certain safety features (*e.g.,* a needle shield) to prevent or minimize sharps injury. Applicant has previously described some embodiments of such safety features in PCT Patent Application WO2022026275 titled SYSTEMS AND METHODS FOR PRE-FILLED MEDICAL DELIVERY ASSEMBLIES. Additional benefits may be achieved by providing additional or alternate safety features for such injection devices. US 2009/171311 A1 proposes a dispenser and therapeutic package suitable for administering a therapeutic substance to a subject. US 2021/0128835 A1 proposes a pre-filled dual-chamber medical delivery agent system. US 2005/0049560 A1 proposes a needle protection assembly. US 2015/0283332 A1 proposes a hinged cap needle assembly.

### SUMMARY

In one embodiment, there is provided a pre-filled medical delivery assembly as set out in claim 1. It has a blow-fill-seal (BFS) bottle with a collapsible reservoir, a neck portion, and a mounting flange formed on the neck portion, a needle hub having a collar with a mounting socket with an interior seat into which the mounting flange is axially mated, and a double-ended needle attached to the needle hub with a piercing end within the mounting socket and an administration end. A cap is removably attached to the needle hub and covers the administration end of the needle. A needle shield covers the needle after use and includes a shield base, a shield element, and a hinge flexibly connecting the shield base and the shield. The needle hub further comprises a fully seated position on the pre-filled medical delivery assembly which causes the piercing end of the double-ended needle to pierce a seal of the BFS bottle.

In another embodiment, there is provided a medical administration assembly as set out in claim 8. It includes a collar having a first end defining a mounting socket comprising an interior seat operable to accept an exterior flange of a blow-fill-seal (BFS) bottle that is axially mated, and a second end defining a hub socket having internal threads. A needle hub includes at least one thread cooperatively mated with the external threads of the collar to a first degree of advancement and is coupled to a double-ended needle disposed through the needle hub. A cap covers an administration end of the needle and includes an interior key to drive the needle hub to advance the at least one external thread of the needle hub to a second degree of advancement with respect to the internal threads of the hub socket of the collar. A needle shield covers the administration end of the needle after use and includes a shield base coupled to the medical administration assembly, a shield element, and a hinge element flexibly connecting the shield base and the shield element. Advancement of the threads to the second degree of advancement causes a piercing end of the double-ended needle to pierce a seal of the collar.

### BRIEF DESCRIPTION OF THE DRAWINGS

An understanding of embodiments described herein and many of the attendant advantages thereof may be readily obtained by reference to the following detailed description when considered with the accompanying drawings, wherein:
FIG. 1A, FIG. 1B, FIG. 1C, FIG. 1D, FIG. 1E, and FIG. 1F are various views of a needle shield for a pre-filled medical delivery assembly according to some embodiments;
FIG. 2A, FIG. 2B, FIG. 2C, FIG. 2D, FIG. 2E, and FIG. 2F are various views of the needle shield for a pre-filled medical delivery assembly coupling according to some embodiments; and
FIG. 3A and FIG. 3B are various views of a pre-filled medical delivery assembly according to some embodiments.

### DETAILED DESCRIPTION

### I. Introduction

Embodiments of the present disclosure provide systems and methods for pre-filled medical delivery assemblies that overcome drawbacks of current delivery devices and methods. For example, the pre-filled medical delivery assemblies of some embodiments may include a Blow-Fill-Seal (BFS) vial or bottle coupled to a specialized collar that facilitates coupling of an administration member (e.g., a needle) to the BFS vial. In some embodiments, such a pre-filled medical delivery assembly may be selectively actuated by application of rotational and/or axial force to a cap covering the administration member, causing the administration member to axially advance and pierce a reservoir of the BFS vial. Utilization of such systems that employ BFS vials may be advantageous and may address various shortcomings of previous systems. In still other embodiments, the collar may be integral with or pre-attached to the administration member before assembling to the BFS vial.

BFS vials may, for example, offer a less expensive alternative to typical vials or devices created via other manufacturing techniques. In some embodiments, BFS vials (*e.g.,* due to the nature of the BFS manufacturing process) may not require separate sterilization (*e.g.,* may accordingly be compatible with a wider array of fluids and/or liquids), may provide enhanced production rates of sterile/aseptic units per hour, and/or may be provided to an end-user for significantly lower per dose/unit costs. In some embodiments, these advantages may come with an attendant drawback of reduced manufacturing tolerances and other disadvantages of utilizing a "soft" plastic (*e.g.,* having a Shore/Durometer "D" hardness of between 60 and 70). BFS processes may, for example, offer less precise manufacturing tolerances in the range of five hundredths of an inch (0.05-in; 1.27 mm) to fifteen hundredths of an inch (0.15-in; 3.81 mm) - for linear dimensions, *e.g.,* in accordance with the standard ISO 2768-1 "General tolerances for linear and angular dimensions without individual tolerance indications" published by the International Organization for Standardization (ISO) of Geneva, Switzerland (November 15, 1989) and/or may not be readily adaptable to form certain mating features such as standardized threads. In some embodiments, these drawbacks and/or the deficiencies of prior systems may be advantageously addressed by specific features, configurations, and/or components as described hereinafter.

Further, in some embodiments it may be desired to dispose of the cap once removed from the needle before use of the pre-filled medical assembly. In such an embodiment, a separate needle shield may be attached to the pre-filled medical assembly and rotated, flipped, and/or otherwise engaged with or on to the needle (*e.g.,* from the side) after use to cover the needle in preparation for disposal.

### II. Pre-filled Medical Delivery Assemblies - Needle Shield attached to Mounting Collar

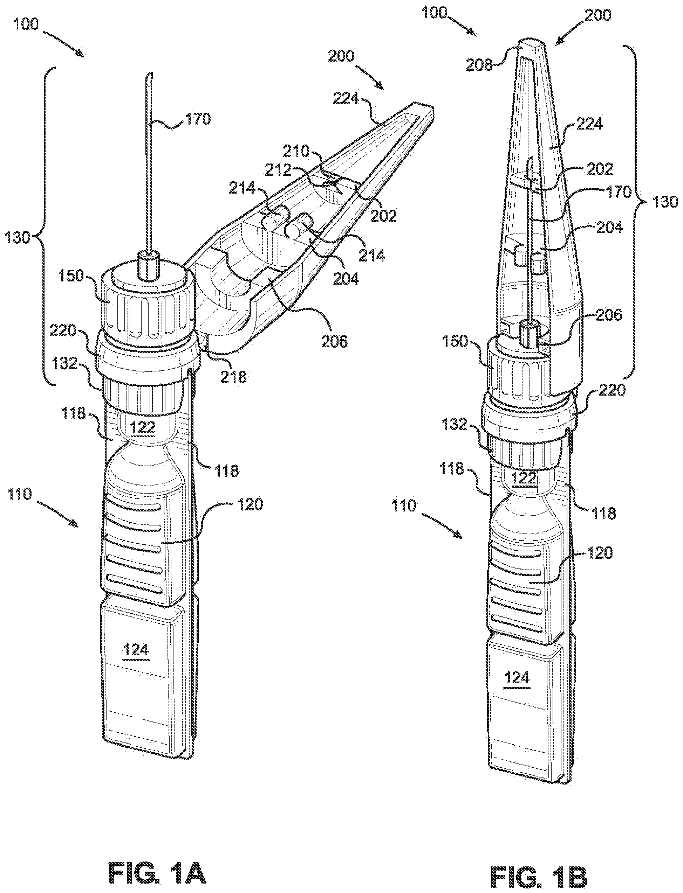

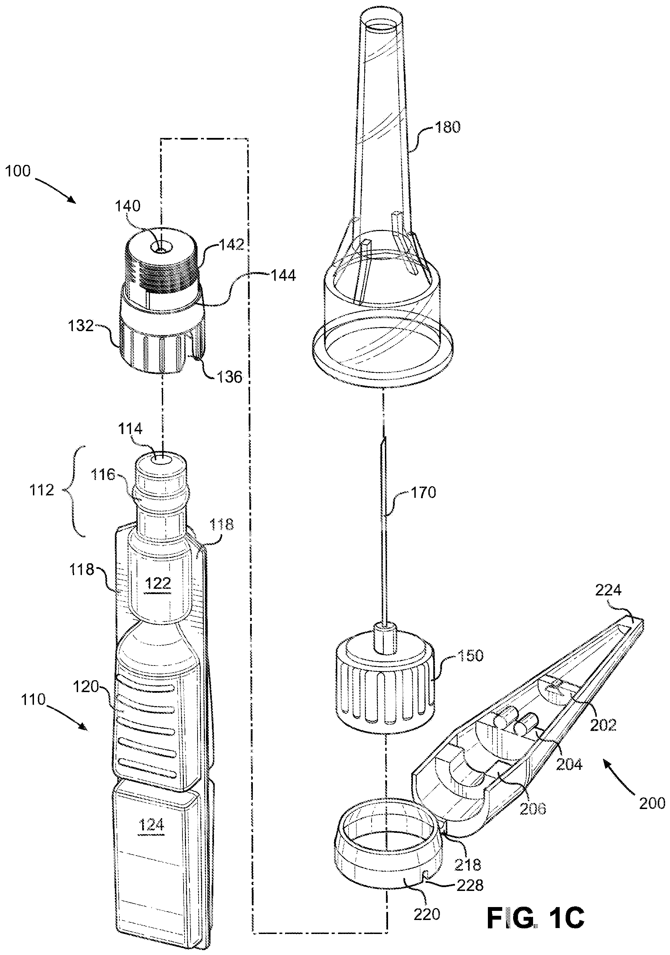

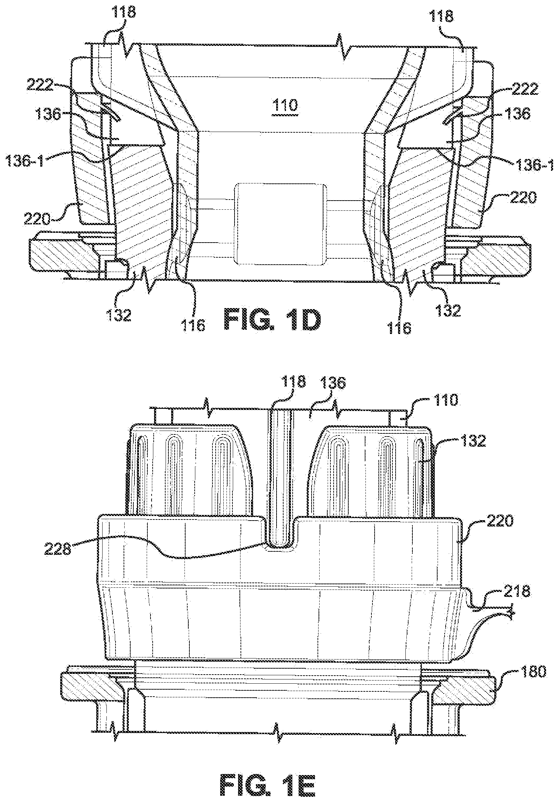

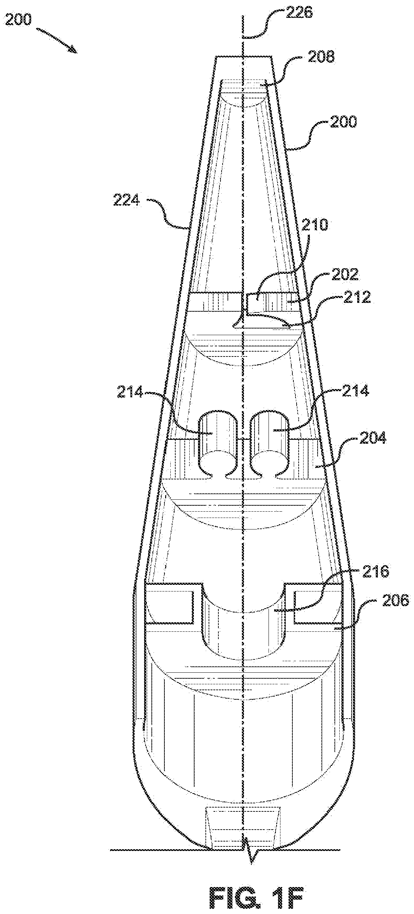

Referring initially to FIG. 1A, FIG. 1B, FIG. 1C, FIG. 1D, FIG. 1E, and FIG. 1F, various views of a pre-filled medical delivery assembly 100 according to some embodiments are shown. In some embodiments, the pre-filled medical delivery assembly 100 may comprise various inter-connected and/or modular components such as a BFS vial 110 comprising and/or defining a vial neck 112, a seal 114, a mounting flange 116, a vial flange 118, a collapsible reservoir 120, a dispensing reservoir 122, and/or an identification area 124. According to some embodiments, the pre-filled medical delivery assembly 100 may comprise an administration (*e.g.,* injection) module or component 130 that is manufactured, assembled, and/or provided as a separate unit from the BFS vial 110. In some embodiments, the administration component 130 may comprise a mounting collar 132 which itself comprises, is coupled to, and/or defines various features and/or elements. The mounting collar 132 and/or the administration assembly 130 may, for example, be maintained as a closed and/or sterile component via a seal 134 (*e.g.,* a foil, wax, paper, and/or other thin, pierceable, tear-able, and/or removable object or layer coupled to the mounting collar 132 and/or the administration component 130; not shown) that seals an interior volume or socket (not separately labeled) of the mounting collar 132, disposed at a first end thereof.

According to some embodiments, the mounting collar 132 may comprise one or more coupling or mounting features such as one or more axial slits or slots 136, an internal seat (not shown) that corresponds to and is the inverse of the mounting flange 116 of the BFS vial 110 to mount the collar 132 to the BFS vial 110 in the case that the neck 112 of the BFS vial 110 is inserted into the mounting collar 132 and/or the administration component 130, a puncture seal 140, and/or external threads 142. It should be known that while external threads 142 are depicted in the figures, the mounting collar 132 could comprise internal threads and/or other mating features without deviating from the scope of the disclosure. In some embodiments, the mounting collar 132 may comprise and/or define an exterior flange 144 (*e.g.,* a radial flange) that is operable to receive, mate with, and/or otherwise engage with a needle hub 150. The mounting collar 132 may include the slot(s) 136 on at least one side. The slot(s) 136 fits over and receives the vial flange 118, preventing the mounting collar 132 from rotating on the BFS vial 110 once attached. In some embodiments, the BFS vial 110 includes two vial flanges 118 on either side of the BFS vial 110 (as depicted), and the mounting collar 132 includes two slots 136 on opposite sides to receive two bottle or vial flanges 118 (*e.g.,* terminal portions thereof; also as depicted).

In some embodiments, the needle hub 150 may couple to the mounting collar 132 via internal threads (within the needle hub 150, not shown) thereof that correspond to and/or mate with the threads 142 of the mounting collar 132. It should be known that while internal threads on the needle hub are described, the needle hub 150 may comprise external threads and/or other mating features that mate with internal threads and/or other corresponding mating features of the mounting collar 132 without deviating from the scope of the disclosure. According to some embodiments, the needle hub 150 may comprise, couple to and/or house a needle, canula, and/or other administration member 170, and/or a cap 180 (*e.g.,* selectively engaged and/or coupled to the needle hub 150 to shroud, house, and/or protect the administration member 170). According to some embodiments, the pre-filled medical delivery assembly 100 may include a modular design consisting of separately constructed components 110, 132, 150, 170, 180 cooperatively arranged and coupled to one another.

In some embodiments, the collapsible reservoir 120 may be filled (fully or partially) with a fluid such as a liquid or other agent (not separately shown) to be delivered, *e.g.,* to a patient (not shown). According to some embodiments, the liquid may be injected into the BFS vial 110 in a sterile environment during manufacture via a BFS process and sealed within the BFS vial 110 via the seal 114. The seal 114 may comprise a portion of the molded BFS vial 110 for example that is configured to be pierced to expel the liquid, *e.g.,* such as by providing a flat or planar piercing surface and/or by being oriented normal to an axis of the BFS vial 110 (and/or the pre-filled medical delivery assembly 100). In some embodiments, the seal 114 may comprise a foil, wax, paper, and/or other thin, pierceable object or layer coupled to the BFS vial 110. In some embodiments, the neck 112 of the BFS vial 110 may comprise the mounting flange 116 such as, *e.g.,* the "doughnut"-shaped exterior flange depicted. The mounting flange 116 may, for example, provide a radially elastic mating surface that is operable to provide a selective engagement or fit within the socket of the administration component 130.

In some embodiments, the administration assembly 130 may include a needle shield 200. The needle shield 200 may include an attachment base 220, which may comprise a ring-shaped, annular, or "c"-clamp style base that fits around the mounting collar 132, but may be any shape to fit around any element of the pre-filled medical delivery assembly 100 such as the BFS bottle 110. In an embodiment, the attachment base 220 includes a flexible locking tab 222 that corresponds to the slot 136 of the mounting collar 132. As depicted in the section view of FIG. 1D, the flexible locking tab 222 fits into a space between the top of the vial flange 118 and the top of the slot 136. The flexible locking tab 222 may be angled such that in use, as the attachment base 220 is slid over the mounting collar 132 and the flexible locking tab 222 is urged outwardly toward the body of the attachment base 220 and minimizes resistance to the sliding movement. Once the flexible locking tab 222 passes the top of the slot 136, it is urged (*e.g.,* by inherent and/or elastic spring and/or otherwise inward radial force) toward the space within the slot 136. According to some embodiments, an interior diameter of the base 220 is less than the width of the bottle flange 118, preventing the attachment base 220 from sliding past the vial flange 118 (up in the orientation of the view of FIG. 1D). If urged in the opposite direction (down in the orientation of FIG. 1D), a tip of the locking tab 222 may interfere with a slot surface 136-1 at the base of the slot 136 and prevent the attachment base 220 from being removed in a direction away from the BFS vial 110.

In still other embodiments, the attachment base 220 may be located on the mounting collar 132 and held in place by the needle hub 150. The attachment base 220 may fit over the threads 142 of the mounting collar 132 and register against the flange 144 of the mounting collar 132. The needle hub 150 may then attach to the mounting collar 132 and capture the attachment base 220 between a bottom portion of the needle hub 150 and the flange 144.

According to some embodiments, a hinge 218 of the needle shield 200 may comprise a thin, flexible hinge that is the same material as the base 220 and a shield element 224, and may be molded with and/or integral to the needle shield 200. This is typically referred to as a "living hinge", and allows the rigid base 220 and shield element 224 to rotate with respect to one another with minimal friction and wear. The living hinge 218 may be described as a purposeful fault line at a predetermined point in the material such that the needle shield 200 doesn't fail after repeated bending between shipping, administration, and covering positions (described below). Molding a living hinge 218 with, between, and/or connecting the attachment base 220 and the shield element 224 of the needle shield 220 reduces manufacturing complexity and assembly tolerance stacks that could lead to mismatches between the needle shield 220 and the administration member 170. Typical types of living hinges include a straight or flat living hinge which is simply a straight, thin portion of the material that provides a convenient are for hinging action, a butterfly living hinge that urges pieces into the open or closed positions, a child safe hinge which includes features designed to make it child resistant, and a double or triple living hinge which include multiple portions of a hinge all working together. The living hinge 218 allows the shield element 224 to fold in an infinite number of positions in about one hundred and eighty (180) degrees with respect to the administration assembly 130. This freedom of placement of the shield element 224 allows the administration assembly 130 to be placed in a shipping position such that the administration assembly may be packaged and shipped in the most convenient and efficient way possible, an administration position whereby the needle shield is conveniently located to allow the administration of the fluid, and a covering position whereby the administration element 170 is captured within the locating/restraining elements 202 and 204, all without the hinge 218 failing and keeping the shield 224 located correctly. While the hinge 118 is depicted as a living hinge, other types, configurations, and/or quantities of hinges may be utilized in some embodiments.

According to some embodiments, the fluid (*e.g.,* a liquid) in the BFS vial 110 may pass between the collapsible reservoir 120 and a connected dispensing reservoir 122. In some embodiments, a juncture, valve, and/or passage (not separately labeled) between the dispensing reservoir 122 and the collapsible reservoir 120 may restrict flow such that the liquid may readily enter one of the dispensing reservoir 122 and the collapsible reservoir 120 but may not readily return to the other reservoir 120, 122. Such a constriction may in some embodiments, provide various advantages in fluid retention and/or administration. In some embodiments, the constriction may not be necessary or desirable, such as in the case that the collapsible reservoir 120 and the dispensing reservoir 122 are formed and/or combined as a single, unobstructed reservoir, e.g., a single reservoir (not shown).

In some embodiments, the pre-filled medical delivery assembly 100 may include a modular design consisting of separately constructed components 110, 130 cooperatively arranged and coupled to one another. As depicted in FIG. 1C, for example, the BFS vial 110 and the administration component 130 may be manufactured, packaged, shipped, stored, and/or provided as separate components. In such a manner, the administration component 130 may not need to be stored or shipped in accordance with often restrictive requirements imposed on medicaments and may accordingly reduce the amount of space required for such specialized storage and/or shipping. The administration component 130 may also or alternatively be manufactured, stored, and/or shipped in advance (*e.g.,* at a first time) while the BFS vial 110 that is pre-filled with the liquid may be manufactured, stored, and/or shipped at a later time (*e.g.,* a second time). In some embodiments, the delay between the first time and the second time may be lengthy without causing determinantal effects, as the administration component 130 may be stored, in some embodiments, indefinitely. In such a manner, units of the administration component 130 may be provided to be on-hand in advance of the availability and/or arrival of the BFS vial 110, reducing supply chain constraints in the case of proactive administration component 130 procurement.

According to some embodiments, the components 110, 130 may be coupled, *e.g.,* in the field and/or in *situ,* to provide an active pre-filled (*e.g.,* injectable) medical delivery device or may be coupled during an automated and/or remote assembly process. The BFS vial 110 and the administration component 130 may be provided as separate units that are coupled together by a user that administers an injection to a patient, for example, or may be pre-assembled and/or coupled to reduce the number of administration steps required for the administration user (*e.g.,* nurse, doctor, patient, etc.). According to some embodiments, the administration component 130 (and/or the mounting collar 132 thereof) may be axially engaged to couple with the BFS vial 110 via application of a mating axial force. The administration component 130 (and/or the mounting collar 132 thereof) may be urged onto the neck 112 of the BFS vial 110, for example, such that the cooperatively shaped internal seat (*e.g.,* an interior groove or channel) accepts the mounting flange 116, thereby removably coupling the BFS vial 110 and the administration component 130 (and/or the mounting collar 132 thereof). In some embodiments, the internal seat (and/or other interior features) and/or the mounting flange 116 may be shaped such that uncoupling of the BFS vial 110 and the administration component 130 (and/or the mounting collar 132 thereof) is mechanically prohibited. In some embodiments, the mounting flange 116 may be shaped as an axially elongated rounded exterior flange (*e.g.,* the "doughnut" shape as depicted) and/or the internal seat may comprise a cooperative and/or mirrored axially elongated rounded interior groove or track. According to some embodiments, the one or more slots 136 such as the mirrored axial slits depicted may engage with the vial flange 118 (and/or portions of the BFS vial 110) such that rotation of the administration component 130 (and/or the mounting collar 132 thereof) with respect to the BFS vial 110 is restricted in the case that they are coupled..

In some embodiments the neck 112 of the BFS vial 110 may be urged and/or forced into the socket of the mounting collar 132 until the mounting flange 116 becomes seated in (and/or coupled to or mated with) the internal seat (*e.g.,* a seated position). In such a manner, the seal 114 may be advantageously positioned adjacent to the puncture seal 140. According to some embodiments, the mounting flange 116 may be configured as the doughnut shape (as depicted) to provide various advantages to the pre-filled medical delivery assembly 100. The axial elongation of the mounting flange 116 may, for example, provide for a smooth, uniform, and/or less forceful mating process that is less likely to deform the soft plastic neck 112 of the BFS vial 110 and/or may provide for a lengthened mating surface that is more likely to prevent leakage of the liquid. In some embodiments, the mounting flange 116 and the cooperatively shaped and sized internal seat (not shown) may permit simple, effective, and/or economic attachment of the administration component 130 to the BFS vial 110.

In some embodiments, the BFS vial 110 and the administration component 130 (and/or the mounting collar 132 thereof) may be axially mated and/or otherwise coupled without utilization of the mounting flange 116. The mounting flange 116 may, for example, not be included or may not be utilized. According to some embodiments, the mating may be effectuated, as depicted in FIG. 1E, be seating and/or engagement of the vial flange 118 within the slot 136. In some embodiments a single slot 136 and vial flange 118 coupling may be utilized, a pair of slots 136 and vial flange 118 couplings (as depicted) may be utilized, or more slots 136 and vial flange 118 couplings may be utilized (*e.g.,* three (3) or more). In some embodiments, the coupling/mating of the slot(s) 136 and vial flange(s) 118 may comprise an interference, tapered, elastic deformation, and/or other fit that securely (and/or removably) couples the BFS vial 110 and the administration component 130 (and/or the mounting collar 132 thereof) - *e.g.,* whether or not the mounting flange 116 is present and/or utilized.

In some embodiments, the needle hub 150 may be coupled to the mounting collar 132 via engagement of the external threads 142 of the mounting collar 132 with the internal threads of the needle hub 150 (and/or otherwise coupled and/or mated). In some embodiments, the internal threads may correspond and cooperate with the external threads 142 such that they may be rotationally and/or removably coupled. According to some embodiments, the administration component 130 may be provided with the mounting collar 132 and the needle hub 150 partially engaged (*e.g.,* with the threads being partially coupled). In some embodiments, the partial engagement (or first engagement state) may cause the administration member 170 (*e.g.,* a second or proximal end thereof) to be positioned adjacent to (and/or in contact with) the puncture seal 140 of the mounting collar 132. In such a first engagement state, the administration component 130 may be coupled to the BFS vial 110, but the BFS vial 110 (and/or the puncture seal 140) have not yet been punctured and/or breached by the administration member 170. In some embodiments, such as in the case that the mating of the BFS vial 110 and the administration component 130 (and/or the mounting collar 132 thereof) is effectuated between the slot(s) 136 and vial flange(s) 118, the slot(s) 136 and vial flange(s) 118 may comprise one or more indexing and/or staging features (*e.g.,* tabs, depressions, protrusions, shaped features, etc.) that provide for the multiple axial engagement states.

According to some embodiments the needle hub 150 may couple to and/or retain the administration member 170. The administration member 170 may be inserted into and/or through the needle hub 150, for example, such that it comprises a first or administration end extending axially distal from the BFS vial 110 and a second or piercing end disposed within the needle hub 150. In some embodiments, the administration end and/or a distal portion of the administration member 170 may be housed, shrouded, and/or covered by the cap 180. According to some embodiments, the cap 180 may be configured to house the administration member 170 and to removably couple to the mounting collar 132 (*e.g.,* by fitting over an external portion thereof and/or by engaging with the external flange 144).

According to some embodiments, the mounting collar 132 and needle hub 150 combination may be utilized to couple and/or mate the administration member 170 with the BFS vial 110 to provide a mechanism via which the administration member 170 may be coupled to the soft plastic BFS vial 110 in a reliable manner. Due to the nature of the BFS plastic and/or process and/or the small form-factor of the BFS vial 110, for example, providing standard external threads (not shown) directly on the neck 112 may not be a viable option for it would result in an imprecise, unreliable, and/or non-water tight coupling (*i.e.,* the threads would be deformable even if they could be properly manufactured to within the desired tolerances, which itself is not a likely result) between he BFS vial 110 and, *e.g.,* the needle hub 150.

In some embodiments, the administration member 170 may include a needle shaped and/or sized for at least one of subcutaneous, intramuscular, intradermal, and intravenous injection of the liquid agent into the patient. For ease of explanation and description, the figures and the description herein generally refer to the administration member 170 as a needle or canula. However, it should be noted that, in other embodiments, the administration member 170 may include a nozzle (not shown) configured to control administration of the liquid agent to the patient. The nozzle may include a spray nozzle, for example, configured to facilitate dispersion of the liquid agent into a spray. Accordingly, a needle hub 150 fitted with a spray nozzle may be particularly useful in the administration of a liquid agent into the nasal passage, for example, or other parts of the body that benefit from a spray application (*e.g.,* ear canal, other orifices). In other embodiments, the nozzle may be configured to facilitate formation of droplets of the liquid agent. Thus, a needle hub 150 including a droplet nozzle may be useful in the administration of a liquid agent by way of droplets, such as administration to the eyes, topical administration, and the like.

As generally understood, the liquid or drug (*e.g.,* stored in the BFS vial 110 and/or one or more of the reservoirs 120, 122 thereof) agent may include any type of agent to be injected into a patient (*e.g.,* animal such as a mammal, either human or non-human) and capable of producing an effect (alone, or in combination with an active ingredient). Accordingly, the agent may include, but is not limited to, a vaccine, a drug, a therapeutic agent, a medicament, a diluent, and/or the like. According to some embodiment, either or both of the liquid agent and the active ingredient (*i.e.,* the drug agent and/or components thereof) may be tracked, monitored, checked for compatibility with each other, etc., such as by utilization of electronic data storage devices (not shown) coupled to the various modules or components such as the BFS vial 110 (*e.g.,* at, on, or in the identification area 124) and/or the administration component 130.

According to some embodiments, the mounting collar 132, the needle hub 150, the needle shield 200, and/or the cap 180 may be composed of a medical grade material. In some embodiments, the mounting collar 132, the needle hub 150, the needle shield 220, and/or the cap 180, may be composed of a thermoplastic polymer or other "hard" plastic (*e.g.,* greater than 80 on the Rockwell "R" scale), including, but not limited to, polybenzimidazole, acrylonitrile butadiene styrene (ABS), polystyrene, polyvinyl chloride, or the like. In some embodiments, the pre-filled medical delivery assembly 100 may be advantageously manufactured (in mass quantities) in separate parts or portions, namely, at least the "soft" plastic BFS vial 110 portion (*e.g.,* a "first" piece) and the "hard" plastic administration component 130 (*e.g.,* the "second" piece), with such different plastic parts/portions being selectively coupled to administer a medication to a patient.

According to some embodiments, the pre-filled medical delivery assembly 100 may be advanced from the first engagement state to a second engagement state where the administration member 170 has pierced the BFS vial 110 and the liquid therein may readily be expressed through the administration member 170 (*e.g.,* and into a patient). The partial engagement of the mounting collar 132 and the needle hub 150 (*e.g.,* with the threads being partially coupled and/or with the slot(s) 136 and vial flange(s) 118 being partially engaged) may, for example, be transitioned to a more advanced, fully, or completely advanced state by application of a rotational (and/or an axial) force to the cap 180. In some embodiments, the more advanced and/or full engagement may cause the administration member 170 (*e.g.,* the second or proximal end thereof) to advance through the puncture seal 140 of the mounting collar 132 and through the seal 114 of the BFS vial 110. In such a manner, for example, the continued rotational engagement of the threads (and/or axial engagement of the administration member 170) may cause the administration member 170 (*e.g.,* the second or proximal end thereof) to open a liquid pathway between the BFS vial 110 (*e.g.,* and/or the neck 112, and/or reservoirs 120, 122 thereof) and the first or distal end of the administration member 170. In some embodiments, the cap 180 may be utilized as a rotational and/or axial force driver to transfer rotational force to the needle hub 150 to cause the advancement of the engagement of the threads (and, *e.g.,* the puncturing of the puncture seal 140 and the seal 114 of the BFS vial 110).

In some embodiments, the administration component 130 may comprise and/or be coupled to the needle shield 200. The needle shield 200 may comprise, for example, the shield base 220 that is mounted on and/or around the mounting collar 132 (and/or the neck 112 of the BFS vial 110). According to some embodiments, the shield base 220 may comprise and/or be coupled to the hinge element 218 and/or may comprise one or more mounting features 202, 204, 206. The hinge element 218 may flexibly couple the shield base 220 to the shield element 224, for example, the shield element 224 comprising a molded and/or shaped element configured to be pushed on the side of the shield element 224 to selectively cover the administration member 170. In some embodiments, the shield element 224 may comprise and/or define a shield volume or space (not separately labeled) that is sized and/or shaped to accept and/or house the administration member 170. In some embodiments such as depicted in FIG. 1F, the shield space may comprise, define, and/or house a first mounting feature or needle keeper 202 (*e.g.,* in the case that the administration member 170 comprises a needle and/or canula), a second mounting feature or locating element 204, and/or a third mounting feature or needle hub keeper 206. In some embodiments, the needle keeper 202 may comprise and/or define a retention barb 210 and/or a retention space 212 that are configured to accept and/or retain a first or distal end of the administration member 170. According to some embodiments, the locating element 204 may comprise and/or define locating barbs 214 that engage with a second portion of the administration member 170 such as to guide the administration member 170 to be in alignment with a shield axis 226. In some embodiments, the needle hub keeper 206 may comprise and/or define a shaped locator element 216 that may, for example, be cooperatively shaped to align a portion of the needle hub 150 with the shield axis 226. In some embodiments, such as depicted in FIG. 1E, the needle shield 200 and/or the shield base 220 thereof may comprise and/or define a seat or notch 228 that aligns with, accepts, and/or mates with a portion (*e.g.,* a terminus and/or extent) of the vial flange 118. In such a manner, for example, the needle shield 200 may be indexed and/or mated with the vial flange 118, thereby aligning and/or coupling the needle shield 200 with/to the BFS vial 110. In some embodiments, the shield base 220 may be restrained and/or retained by being sandwiched between the vial flange 118 seated in the notch 228 (which prevents axial movement in a first direction) and the needle hub 250 (that prevents axial movement in a second and opposite direction). According to some embodiments, the pre-filled medical delivery assembly 100 may include a modular design consisting of separately constructed components 110, 130, 132, 150, 170, 180, 200 cooperatively arranged and coupled to one another.

In some embodiments, fewer or more components 110, 112, 114, 116, 118, 120, 122, 124, 130, 132, 136, 140, 142, 144, 150, 170, 180, 200, 202, 204, 206, 208, 210, 212, 214, 216, 218, 220, 222, 224, 226, 228 and/or various configurations of the depicted components 110, 112, 114, 116, 118, 120, 122, 124, 130, 132, 136, 140, 142,144, 150, 170, 180, 200, 202, 204, 206, 208, 210, 212, 214, 216, 218, 220, 222, 224, 226, 228 may be included in the pre-filled medical delivery assembly 100 without deviating from the scope of embodiments described herein. In some embodiments, the components 110, 112, 114, 116, 118, 120, 122, 124, 130, 132, 136, 140, 142, 144, 150, 170, 180, 200, 202, 204, 206, 208, 210, 212, 214, 216, 218, 220, 222, 224, 226, 228 may be similar in configuration and/or functionality to similarly named and/or numbered components as described herein. According to some embodiments, the pre-filled medical delivery assembly 100 may comprise the mounting flange 116 but not the collapsible reservoir 120. In some embodiments, the pre-filled medical delivery assembly 100 may comprise the mounting flange 116 but not the dispensing reservoir 122. According to some embodiments, the administration component 130 may be provided without (and/or separately from) the BFS vial 110. In some embodiments, the puncture seal 140 may not be included and/or the mounting collar 132 and the needle hub 150 may comprise an integrated and/or unitary molded and/or constructed element. In some embodiments, the needle shield 200 may be integrated with the mounting collar 132, the needle hub 150, and/or otherwise with the administration component 130.

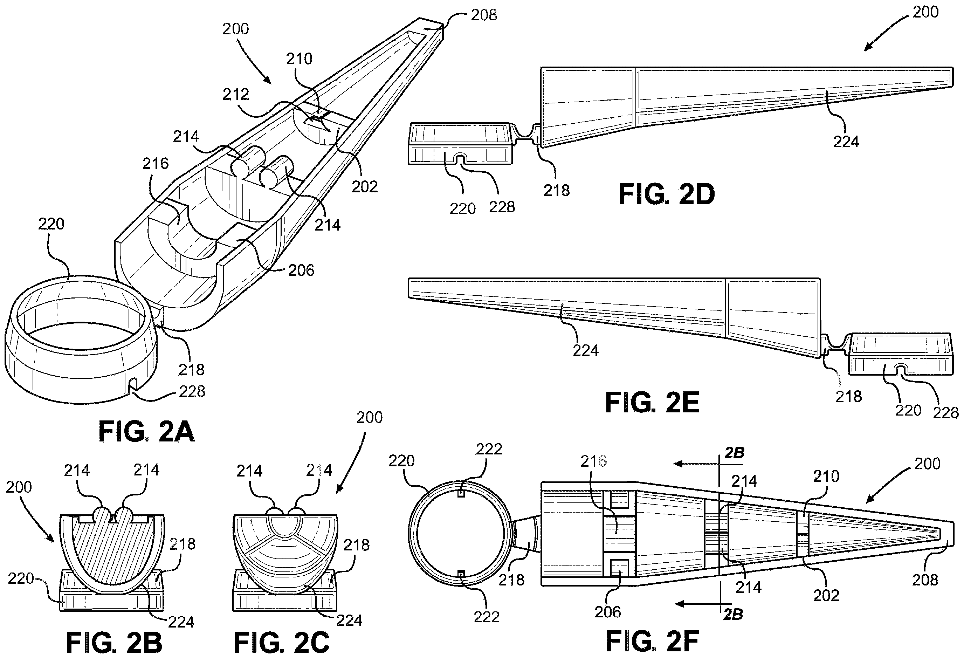

Referring now to FIG. 2A, 2B, 2C, 2D, 2E, and 2F, in some embodiments, a needle shield 200 may include any or all of three locating/restraining elements 202, 204, 206 disposed within and/or formed in a shield element 224 that is coupled to a shield base 220. A locating/restraining element or retention member 202 may, in some embodiments, be configured to couple to, restrain, and/or accept a canula/needle (not shown). A second locating/restraining element or locating element 204 may comprise a feature that facilitates alignment of the canula/needle within the needle shield 200.A third locating element 206 may be a coarse locator that includes a half-cylinder locator 216 that fits around the needle hub 150 (*e.g.,* a portion thereof) and ensures that the needle shield 200 is located properly with respect to the length of the canula/needle (*e.g.,* the administration member 170 of FIG. 1C herein) and that a tip 208 fits over the top (*e.g.,* a piercing end or point) of the canula/needle. According to some embodiments, the locating element 204 is located between the locating/restraining elements 202 and 206 and may include a pair of locating barbs 214. The locating barbs 214 may be centered along a shield axis (not shown; *e.g.,* the shield axis 226 of FIG. 1F herein) to capture an intermediate portion of the canula/needle and locate the needle shield 200 more finely than the locating element 206. The barbs 214 may be placed and/or configured (*e.g.,* as axial cylindrical elements as shown) such that the canula/needle easily fits within the barbs 214 but requires some force to remove the canula/needle. The retention member 202 may include a retention barb 210 and a retention space 212. The retention barb 210 may be shaped such that the canula/needle is able to easily slide past the retention barb 210 and into the retention space 212, but removal of the canula/needle requires more force to push the retention barb 210 back upon itself to open and allow the canula/needle to release from the retention space 212. According to some embodiments, the needle shield 200 and/or the . In some embodiments, the needle shield 200 and/or the shield base 220 thereof may comprise one or more locking tabs 222 disposed on and/or projecting radially inward from an interior surface of the shield base 220. According to some embodiments, the needle shield 200 and/or the shield base 220 thereof may comprise and/or define one or more notches 228 disposed and/or formed in a radial circumference of the shield base 220. In some embodiments, the notch(es) 228 may be radially aligned with the one or more locking tabs 222.

In some embodiments, fewer or more components 202, 204, 206, 208, 210, 212, 214, 216, 218, 220, 222, 224, 228 and/or various configurations of the depicted components 202, 204, 206, 208, 210, 212, 214, 216, 218, 220, 222, 224, 228 may be included in the needle shield 200 without deviating from the scope of embodiments described herein. In some embodiments, the components shield base 220 may attach or couple to a BFS bottle (not shown; *e.g.,* the BFS vial 110 of FIG. 1A herein). Some embodiments may include the restraining element 202 but not the locating/restraining elements 204 and 206. Some embodiments may include the locating/restraining element 204 but not the locating/restraining elements 202 and 206. In some embodiments, the needle shield 200 is provided as a separate element from an administration component (not shown; *e.g.,* the administration component 130 of FIG. 1A herein) and attaches to a pre-filled medical delivery assembly as a third module thereof. In these embodiments, the BFS bottle and the administration component may be assembled to one another independently, and the needle shield 200 may be coupled to the medical delivery assembly by either sliding the shield base 220 up over the BFS bottle or over the top of a cap thereof, into its coupled position.

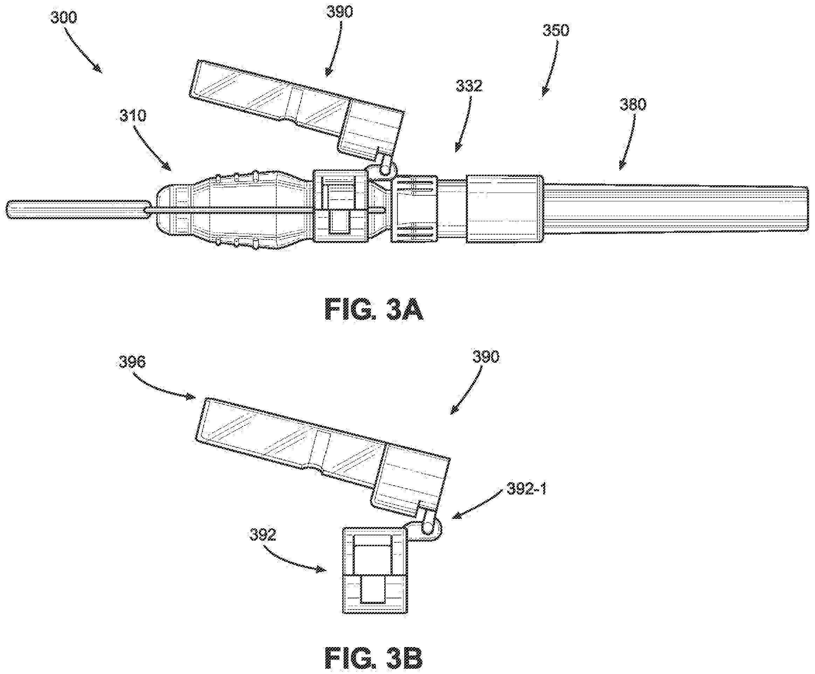

Referring now to FIG. 3A and FIG. 3B, side and partial assembly views of a pre-filled medical delivery assembly 300 according to some embodiments are shown. The pre-filled medical delivery assembly 300 may comprise similar features and/or configurations and/or may be similar to the other assemblies described herein. The pre-filled medical delivery assembly 300 may comprise and/or define, for example, a BFS vial 310 coupled to an administration assembly 350 (comprising a mounting collar 332 and cap 380), and/or a needle shield 390. In some embodiments, the needle shield 390 may comprise and/or define a shield base 392, a hinge element 392-1, and/or a shield element 396 (*e.g.,* defining and/or comprising a shield space; not separately labeled). In some embodiments, the shield base 392 may comprise a circular and/or annular ring or collar through which a portion of the BFS vial 310 may pass and/or be mounted. According to some embodiments, the shield base 392 may be sized and/or shaped to snap onto and/or house (i) a neck of the BFS vial 310 (not separately labeled) (ii) a portion of the BFS vial 310 disposed between a compressible chamber of the BFS vial 310 (not separately labeled) and a neck of the BFS vial 310, and/or (iii) the administration assembly 350. In some embodiments, the shield base 392 may be sized and/or shaped to snap onto and/or house the mounting collar 332 (*e.g.,* similar to the pre-filled medical delivery assembly 100 and the needle shield 220 of FIG. 1A, FIG. 1B, FIG. 1C, FIG. 1D, FIG. 1E, and FIG. 1F herein).

In such a manner, for example, the shield base 392 may be selectively and/or removably coupled to the BFS vial 310 and/or the mounting collar 332. According to some embodiments, the shield base 392 may be coupled to and/or comprise the hinge element 392-1 that may connect and/or link the shield base 392 to the shield element 396. In such a manner, for example, in the case that the shield base 392 is coupled to and/or mounted on the BFS vial 310 (and/or the mounting collar 332), the shield element 396, by virtue of the connection via the hinge element 392-1 may also be coupled to the BFS vial 310. The hinge element 392-1 may comprise any type, configuration, and/or quantity of hinge and/or elastic connector between the shield base 392 and the shield element 396 that permits the shield base 392 and the shield element 396 to be selectively positioned in a plurality of different orientations with respect to each other. In the case where the hinge element 392-1 comprises a pin and socket hinge (*e.g.,* as shown), for example, the hinge element 392-1 may comprise a first pin and/or socket attached to (and/or integral to) the respective shield base 392 and a second and corresponding socket and/or pin attached to (and/or integral to) the shield element 396 and may permit repositioning about one or more of such elements (*e.g.,* about an axis of connection thereof; not separately labeled).

According to some embodiments, the shield base 392 may be integrated with (or into) the mounting collar 332. The mounting collar 332 may, for example, comprise and/or define the hinge element 392-1 that rotatably couples to the shield element 396. In some embodiments, the shield base 392 may comprise and/or define one or mounting features (*e.g.,* tabs, sockets, projections, detents, apertures, etc.; not separately labeled) that are configured to mate with corresponding mounting features (not shown) of one or more of the BFS vial 310 and the mounting collar 332.

In some embodiments, the coupling of the BFS vial 310 and the administration assembly 350 (and/or the mounting collar 332 thereof) may be configured to explicitly permit free rotation (*e.g.,* about a common axis) of the BFS vial 310 with respect to the administration assembly 350 (and/or the mounting collar 332 thereof).

In some embodiments, fewer or more components 310, 332, 350, 380, 390, 392, 392-1, 396 and/or various configurations of the depicted components 310, 332, 350, 380, 390, 392, 392-1, 396 may be included in the pre-filled medical delivery assembly 300 without deviating from the scope of embodiments described herein. In some embodiments, the components 310, 332, 350, 380, 390, 392, 392-1, 396 may be similar in configuration and/or functionality to similarly named and/or numbered components as described herein.

### III. Pre-filled Medical Delivery Methods

In some embodiments, various methods and/or processes may be performed and/or implemented to utilize a BFS vial and/or bottle filled with a single dose of medicament to administer the single dose to a patient/target. In some embodiments, a method may cause a BFS vial to be coupled to an administration component that is engaged to puncture the BFS vial and then may be utilized to inject (or otherwise administer) the medicament to the patient/target. The methods and/or processes (and/or diagrams and/or flow diagrams thereof) described herein do not necessarily imply a fixed order to any depicted actions, steps, and/or procedures, and embodiments may generally be performed in any order that is practicable unless otherwise and specifically noted. While the order of actions, steps, and/or procedures described herein is generally not fixed, in some embodiments, actions, steps, and/or procedures may be specifically performed in the order listed, depicted, and/or described and/or may be performed in response to any previously listed, depicted, and/or described action, step, and/or procedure.

In practice, for example, some or all of the following procedures may be followed to utilize a pre-filled medical delivery assembly to administer a medication (and/or other liquid) to a patient/target. In some embodiments, the area of injection may be cleaned and/or otherwise prepared. A neck and/or a seal of the BFS vial (*e.g.,* a "first" part and/or component) may be cleaned (*e.g.,* utilizing an alcohol wipe) to prepare the BFS vial for coupling to an administration component and/or assembly. In some embodiments, a "second" part and/or component comprising a pre-packaged mounting collar/coupling, needle hub (with an administration member), cap, and/or needle shield may comprise a seal that maintains an internal volume/liquid passage in a sterile state and the seal may be removed to prepare for the coupling to the BFS vial. According to some embodiments, the administration assembly may be axially aligned with the neck of the BFS vial and the neck may be inserted into the mounting collar and/or through a base member of the needle shield to engage a mounting flange with a cooperatively shaped interior mating feature. According to some embodiments, the administration component (and/or the mounting collar and/or the needle shield thereof) may "click" or snap onto the BFS vial.

According to some embodiments, the needle hub may, as a component of a pre-packaged "second" part/administration assembly for example, be only partially engaged with (*e.g.,* partially threaded onto) the mounting collar. Only a portion of the threads may be engaged, for example, such that the "second" part is at least loosely or partially coupled as a single object/assembly but the administration member is not advanced axially enough to pierce the BFS vial in the case that the "second" part/assembly is mated with the BFS vial. In such a manner, for example, a user may couple the "first" and "second" parts and then selectively engage the administration member to puncture the BFS vial (*e.g.,* by application of rotational force to advance the threads/mating thereof and/or by application of axial force that causes the administration member to pierce the BFS vial).

In some embodiments, a user may hold the mounting collar with one hand/fingers and thread (*e.g.,* continue threading) the needle hub fully into the mounting collar by applying rotational force to the cap. The cap may comprise an internal key that engages with the one or more stop and/or drive features of the needle hub, for example, to transfer the rotational force to the needle hub and accordingly advance the mating of the threads. As the threading/mating advances, a second or piercing end of the administration member may be axially advanced to pierce through the mounting collar (*e.g.,* a seal portion thereof) and/or through the seal of the BFS vial, thereby activating the pre-filled medical delivery assembly. In some embodiments, the stop/drive features may be shaped to comprise one or more surfaces that engage with the internal key of the cap in the case that the cap is rotated in a clockwise direction but may comprise an opposing surface or feature that permits (and/or forces) the internal key to disengage with the stop/drive features in the case that the cap is rotated in a counter-clockwise direction. In such a manner, for example, a user may readily couple (or complete the coupling of) the needle hub and the mounting collar but may be prevented from utilizing the cap to unscrew or decouple the needle hub and the mounting collar. In some embodiments, the internal key may be configured to accept a threshold amount of torque before a designed failure so that the user may readily tighten the threads but any attempt to overtighten will result in separation or destruction of the internal key, thereby preventing the cap from further functioning as a driver of the mating process and accordingly preventing overtightening of the mating between the needle hub and the mounting collar. In some embodiments, an audible "click" or other sound of the failure of the internal key may comprise a designed indication to the user that the pre-filled medical delivery assembly is properly and/o fully assembled/activated. In some embodiments, the user may also or alternatively hold the mounting collar with one hand/fingers and push (*e.g.,* axially) the mounting collar onto the BFS vial, causing the administration member to pierce the BFS vial axially (*e.g.,* with or without rotation).

According to some embodiments, the cap may be removed to reveal the administration member and/or a first or administration end thereof. In some embodiments, the administration member (*e.g.,* the administration end thereof) may be inserted into the patient and a collapsible reservoir of the BFS vial may be squeezed (*e.g.,* receive an application of radially inward force), thereby expelling the liquid through the administration member and into the patient. In some embodiments, the administration member may be withdrawn from the patient and/or a needle shield (*e.g.,* coupled to and/or part of the administration component/assembly) may be selectively moved (*e.g.,* flipped and/or rotated) into position to cover the administration member/needle. As depicted herein, for example, a needle shield that is coupled to either or both of the BFS vial and the administration component/assembly may be selectively flipped up (*e.g.,* from a first, transport, or storage position) to cover, retain, and/or mate with the administration member/needle (*e.g.,* in a second or safety position). According to some embodiments, the first position may be between one hundred and eighty degrees (180°) and ninety degrees (90°) offset from the second position (*e.g.,* along a particular axis and/or within a particular plane). The pre-filled medical delivery assembly may then be properly disposed of. While the mounting collar and the needle hub are generally described and depicted as separate couplable objects, in some embodiments they may be manufactured (*e.g.,* molded) as a single object or piece or may comprise additional pieces or parts.

### IV. Additional Embodiments

In some embodiments, a needle shield may comprise a support base defining a passage therethrough and comprising one or more protrusions within the passage, such protrusions extending radially inward from an inside surface of the support base. According to some embodiments, the support base may also or alternatively comprise one or more notches disposed on a radial circumference thereof. In some embodiments, the one or more notches may align radially (e.g., be positioned at the same points along the circumference) with the one or more protrusions. According to some embodiments, the support base may be coupled to and/or comprise a hinge element that couples the support base to a shield element comprising and/or defining an elongated voided and/or space. In some embodiments, the shield element may comprise a first alignment feature formed within the space at a first distance from the hinge, a second alignment feature formed within the space at a second distance from the hinge, and/or a third alignment feature formed within the space at a third distance from the hinge. According to some embodiments, the first alignment feature may comprise a shaped protrusion that is shaped to accept and/or mate with a portion of a needle hub. In some embodiments, the second alignment feature may comprise a pair of shaped protrusions that are configured to accept a first portion of a needle and guide the first portion of the needle to be in alignment with a central axis of the shield element. According to some embodiments, the third alignment feature may comprise a catch feature that is configured to accept and retain a second portion of the needle (and/or guide the second portion of the needle to be in alignment with the central axis of the shield element). In some embodiments, the pair of shaped protrusions of the second alignment feature may comprise a pair of cylindrical and/or rounded protrusions that are curved toward the central axis. According to some embodiments the hinge may comprise a living hinge. According to some embodiments, the notch may be shaped and/or configured to accept and/or retain a flange of a BFS vial.

### V. Rules of Interpretation

Throughout the description herein and unless otherwise specified, the following terms may include and/or encompass the example meanings provided. These terms and illustrative example meanings are provided to clarify the language selected to describe embodiments both in the specification and in the appended claims, and accordingly, are not intended to be generally limiting. While not generally limiting and while not limiting for all described embodiments, in some embodiments, the terms are specifically limited to the example definitions and/or examples provided. Other terms are defined throughout the present description.

Numerous embodiments are described in this patent application, and are presented for illustrative purposes only. The described embodiments are not, and are not intended to be, limiting in any sense. The presently disclosed invention(s) are widely applicable to numerous embodiments, as is readily apparent from the disclosure. One of ordinary skill in the art will recognize that the disclosed invention(s) may be practiced with various modifications and alterations, such as structural, logical, software, and electrical modifications. Although particular features of the disclosed invention(s) may be described with reference to one or more particular embodiments and/or drawings, it should be understood that such features are not limited to usage in the one or more particular embodiments or drawings with reference to which they are described, unless expressly specified otherwise.

Devices that are in communication with each other need not be in continuous communication with each other, unless expressly specified otherwise.

A description of an embodiment with several components or features does not imply that all or even any of such components and/or features are required. On the contrary, a variety of optional components are described to illustrate the wide variety of possible embodiments of the present invention(s). Unless otherwise specified explicitly, no component and/or feature is essential or required.

Further, although process steps, algorithms or the like may be described in a sequential order, such processes may be configured to work in different orders. In other words, any sequence or order of steps that may be explicitly described does not necessarily indicate a requirement that the steps be performed in that order. The steps of processes described herein may be performed in any order practical. Further, some steps may be performed simultaneously despite being described or implied as occurring non-simultaneously (e.g., because one step is described after the other step). Moreover, the illustration of a process by its depiction in a drawing does not imply that the illustrated process is exclusive of other variations and modifications thereto, does not imply that the illustrated process or any of its steps are necessary to the invention, and does not imply that the illustrated process is preferred.

The present disclosure provides, to one of ordinary skill in the art, an enabling description of several embodiments and/or inventions. Some of these embodiments and/or inventions may not be claimed in the present application, but may nevertheless be claimed in one or more continuing applications that claim the benefit of priority of the present application. Applicants intend to file additional applications to pursue patents for subject matter that has been disclosed and enabled but not claimed in the present application.

It will be understood that various modifications can be made to the embodiments of the present disclosure herein without departing from the scope thereof. Therefore, the above description should not be construed as limiting the disclosure, but merely as embodiments thereof. Those skilled in the art will envision other modifications within the scope of the invention as defined by the claims appended hereto.

While several embodiments of the present disclosure have been described and illustrated herein, those of ordinary skill in the art will readily envision a variety of other means and/or structures for performing the functions and/or obtaining the results and/or one or more of the advantages described herein, and each of such variations and/or modifications is deemed to be within the scope of the present disclosure. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present disclosure is/are used.

Those skilled in the art will recognize, or be able to ascertain using no more than routine experimentation, many equivalents to the specific embodiments of the disclosure described herein. It is, therefore, to be understood that the foregoing embodiments are presented by way of example only and that, within the scope of the appended claims. The present disclosure is directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the scope of the present disclosure.

All definitions, as defined and used herein, should be understood to control over dictionary definitions and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Other elements may optionally be present other than the elements specifically identified by the "and/or" clause, whether related or unrelated to those elements specifically identified, unless clearly indicated to the contrary.

Reference throughout this specification to "one embodiment" or "an embodiment" means that a particular feature, structure, or characteristic described in connection with the embodiment is included in at least one embodiment. Thus, appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

## Claims

1. A pre-filled medical delivery assembly (100, 300), comprising:
a blow-fill-seal (BFS) vial (110) defining a collapsible reservoir (120), a neck portion (112), and at least one vial flange (118) formed adjacent to the neck portion;
a mounting collar (132) comprising (i) a first end comprising a mounting socket comprising an interior seat into which the neck portion of the BFS vial is inserted, (ii) a second end comprising a collar mating feature, and (iii) at least one axial slot (136) extending axially from the first end and toward the second end;
a needle hub (150) comprising at least one hub mating feature cooperatively mated with the collar mating feature;
a double-ended needle (170) disposed through and coupled to the needle hub and comprising a piercing end housed within the needle hub and an administration end distal from the needle hub;
a cap (180) removably attached to the needle hub and covering the administration end of the needle; and
a needle shield (200) comprising a shield base (220) coupled to the mounting collar, a shield element (224), and a hinge element (218) flexibly connecting the shield base and the shield element, wherein the shield element comprises a retention element having a retention barb configured to capture the administration end of the double-ended needle within a retention space of the retention element, and wherein the shield base comprises at least one internal radial protrusion that engages with the at least one axial slot.

2. The pre-filled medical delivery assembly of claim 1, wherein the shield base (220) comprises an annular element through which the mounting collar (132) is inserted.

3. The pre-filled medical delivery assembly of claim 1, wherein the shield base (220) further comprises at least one circumferential notch (228) that couples to the at least one vial flange (118).

4. The pre-filled medical delivery assembly of claim 1, wherein the at least one vial flange (118) is disposed within the at least one axial slot (136).

5. The pre-filled medical delivery assembly of claim 1, wherein the shield element (224) of the needle shield (200) further comprises a locating element (202, 204) disposed further from the administration end of the double-ended needle (170) than the retention barb, the locating element having a pair of locating barbs configured to capture an intermediate portion of the double-ended needle.

6. The pre-filled medical delivery assembly of claim 1, wherein the shield base (220) is retained between the at least one vial flange (118) and the needle hub (150).

7. The pre-filled medical delivery assembly of claim 1, wherein the collar (132) is integral with the needle hub (150).

8. A medical administration assembly, comprising:
a collar (132) having a first end defining a mounting socket comprising an interior seat operable to accept an exterior flange of a blow-fill-seal (BFS) vial (110) that is axially mated, and a second end defining a hub socket comprising a collar mating feature;
a needle hub (150) comprising at least one hub mating feature cooperatively mated with the collar mating feature, the needle hub being coupled to a double-ended needle disposed through the needle hub;
a needle shield (200) comprising a shield base (220) coupled to the collar (132), a shield element (224), and a hinge element (218) flexibly connecting the shield base and the shield element, wherein the shield element comprises a retention element having a retention barb configured to capture the administration end of the double-ended needle within a retention space of the retention element, and wherein the shield base further comprises at least one circumferential notch that can couple to at least one vial flange of the BFS vial.

9. The medical administration assembly of claim 8, wherein the shield element (224) is rotated about the hinge element and coupled with the administration end of the needle after the cap is removed.

10. The medical administration assembly of claim 8, further comprising a removable needle hub seal covering an opening of the mounting socket.

11. The medical administration assembly of claim 8, wherein the collar (132) is integral with the needle hub.

## Patentansprüche

1. Vorbefüllte medizinische Abgabeanordnung (100, 300), umfassend:
eine Blow-Fill-Seal-(BFS)-Ampulle (110), die einen kollabierbaren Behälter (120), einen Halsabschnitt (112) und zumindest einen Ampullenflansch (118), der benachbart zum Halsabschnitt ausgebildet ist, definiert;
einen Befestigungskragen (132), umfassend (i) ein erstes Ende, das eine Befestigungsbuchse umfasst, welche einen Innensitz umfasst, in den der Halsabschnitt der BFS-Ampulle eingebracht ist, (ii) ein zweites Ende, das ein Kragenzusammengriffsmerkmal umfasst, und (iii) zumindest einen axialen Schlitz (136), der sich axial vom ersten Ende aus hin zum zweiten Ende erstreckt;
einen Nadelansatz (150), der zumindest ein Ansatzzusammengriffsmerkmal umfasst, das zusammenwirkend mit dem Kragenzusammengriffsmerkmal in Eingriff gebracht ist;
eine doppelendige Nadel (170), die durch den Nadelansatz hindurch angeordnet und mit diesem gekoppelt ist und ein Einstechende, das innerhalb des Nadelansatzes eingehaust ist, und ein Verabreichungsende distal vom Nadelansatz umfasst;
einen Deckel (180), der entfernbar auf dem Nadelansatz befestigt ist und das Verabreichungsende der Nadel bedeckt; und
eine Nadelschutzkappe (200), umfassend eine mit dem Befestigungskragen gekoppelte Schutzkappenbasis (220), ein Schutzkappenelement (224) und ein Gelenkelement (218), welches die Schutzkappenbasis und das Schutzkappenelement flexibel miteinander verbindet, wobei das Schutzkappenelement ein Rückhalteelement umfasst, welches einen Rückhaltewiderhaken aufweist, der dazu ausgelegt ist, das Verabreichungsende der doppelendigen Nadel innerhalb eines Rückhalteraums des Rückhalteelements zu umgreifen, und wobei die Schutzkappenbasis zumindest einen inneren radialen Vorsprung umfasst, der mit dem zumindest einen axialen Schlitz in Eingriff steht.

2. Vorbefüllte medizinische Abgabeanordnung nach Anspruch 1, wobei die Schutzkappenbasis (220) ein ringförmiges Element umfasst, über das der Befestigungskragen (132) eingebracht ist.

3. Vorbefüllte medizinische Abgabeanordnung nach Anspruch 1, wobei die Schutzkappenbasis (220) ferner zumindest eine Umfangseinkerbung (228) umfasst, die mit dem zumindest einen Ampullenflansch (118) gekoppelt ist.

4. Vorbefüllte medizinische Abgabeanordnung nach Anspruch 1, wobei der zumindest eine Ampullenflansch (118) innerhalb des zumindest einen axialen Schlitzes (136) angeordnet ist.

5. Vorbefüllte medizinische Abgabeanordnung nach Anspruch 1, wobei das Schutzkappenelement (224) der Nadelschutzkappe (200) ferner ein Platzierungselement (202, 204) umfasst, das weiter weg vom Verabreichungsende der doppelendigen Nadel (170) angeordnet ist als der Rückhaltewiderhaken, wobei das Platzierungselement ein Paar von Platzierungswiderhaken aufweist, die dazu ausgelegt sind, einen Mittelabschnitt der doppelendigen Nadel zu umgreifen.

6. Vorbefüllte medizinische Abgabeanordnung nach Anspruch 1, wobei die Schutzkappenbasis (220) zwischen dem zumindest einen Ampullenflansch (118) und dem Nadelansatz (150) gehalten ist.

7. Vorbefüllte medizinische Abgabeanordnung nach Anspruch 1, wobei der Kragen (132) mit dem Nadelansatz (150) einstückig ausgebildet ist.

8. Medizinische Abgabeanordnung, umfassend:
einen Kragen (132) mit einem ersten Ende, das eine Befestigungsbuchse definiert, welche einen Innensitz umfasst, der betätigbar ist, um einen äußeren Flansch einer Blow-Fill-Seal-(BFS-)-Ampulle (110), die axial in Zusammengriff gebracht ist, aufzunehmen, und einem zweiten Ende, das eine Ansatzbuchse definiert, welche ein Kragenzusammengriffsmerkmal umfasst;
einen Nadelansatz (150), der zumindest ein Ansatzzusammengriffsmerkmal umfasst, welches zusammenwirkend mit dem Kragenzusammengriffsmerkmal in Zusammengriff gebracht ist, wobei der Nadelansatz mit einer doppelendigen Nadel gekoppelt ist, die durch den Nadelansatz hindurch angeordnet ist;
eine Nadelschutzkappe (200), umfassend eine mit dem Kragen (132) gekoppelte Schutzkappenbasis (220), ein Schutzkappenelement (224) und ein Gelenkelement (218), welches die Schutzkappenbasis und das Schutzkappenelement flexibel miteinander verbindet, wobei das Schutzkappenelement ein Rückhalteelement umfasst, welches einen Rückhaltewiderhaken aufweist, der dazu ausgelegt ist, das Verabreichungsende der doppelendigen Nadel innerhalb eines Rückhalteraums des Rückhalteelements zu umgreifen, und wobei die Schutzkappenbasis zumindest eine Umfangseinkerbung umfasst, die mit zumindest einem Ampullenflansch der BFS-Ampulle gekoppelt sein kann.

9. Medizinische Abgabeanordnung nach Anspruch 8, wobei das Schutzkappenelement (224) um das Gelenkelement gedreht wird und mit dem Verabreichungsende der Nadel gekoppelt ist, nachdem der Deckel entfernt wurde.

10. Medizinische Abgabeanordnung nach Anspruch 8, ferner umfassend eine entfernbare Nadelansatzversiegelung, die eine Öffnung der Befestigungsbuchse bedeckt.

11. Medizinische Abgabeanordnung nach Anspruch 8, wobei der Kragen (132) mit dem Nadelansatz einstückig ausgebildet ist.

## Revendications

1. Ensemble de distribution médicale pré-rempli (100, 300), comprenant :
un flacon (110) à joint de remplissage par soufflage (BFS) définissant un réservoir pliable (120), une partie de col (112), et au moins une bride de flacon (118) formée adjacente à la partie de col ;
un collier de montage (132) comprenant (i) une première extrémité comprenant une douille de montage comprenant un siège intérieur dans lequel la partie de col du flacon BFS est insérée, (ii) une seconde extrémité comprenant une caractéristique d'appariement de collier, et (iii) au moins une fente axiale (136) s'étendant axialement à partir de la première extrémité et vers la seconde extrémité ;
un moyeu d'aiguille (150) comprenant au moins une caractéristique d'appariement de moyeu appariée en coopération avec la caractéristique d'appariement de collier ;
une aiguille à double extrémité (170) disposée à travers et couplée au moyeu d'aiguille et comprenant une extrémité de perçage logée à l'intérieur du moyeu d'aiguille et une extrémité d'administration distale par rapport au moyeu d'aiguille ;
un capuchon (180) fixé de manière amovible au moyeu d'aiguille et recouvrant l'extrémité d'administration de l'aiguille ; et
une protection d'aiguille (200) comprenant une base de protection (220) couplée au collier de montage, un élément de protection (224) et un élément d'articulation (218) reliant de manière flexible la base de protection et l'élément de protection, dans lequel l'élément de protection comprend un élément de rétention présentant une barbe de rétention configurée pour capturer l'extrémité d'administration de l'aiguille à double extrémité dans un espace de rétention de l'élément de rétention, et dans lequel la base de protection comprend au moins une saillie radiale interne qui vient en prise avec la au moins une fente axiale.

2. Ensemble de distribution médicale pré-rempli selon la revendication 1, dans lequel la base de protection (220) comprend un élément annulaire à travers lequel le collier de montage (132) est inséré.

3. Ensemble de distribution médicale pré-rempli selon la revendication 1, dans lequel la base de protection (220) comprend en outre au moins une encoche circonférentielle (228) qui se couple à la au moins une bride de flacon (118).

4. Ensemble de distribution médicale pré-rempli selon la revendication 1, dans lequel la au moins une bride de flacon (118) est disposée à l'intérieur de la au moins une fente axiale (136).

5. Ensemble de distribution médicale pré-rempli selon la revendication 1, dans lequel l'élément de protection (224) de la protection d'aiguille (200) comprend en outre un élément de positionnement (202, 204) disposé plus loin de l'extrémité d'administration de l'aiguille à double extrémité (170) que la barbe de retenue, l'élément de positionnement présentant une paire de barbes de positionnement configurées pour capturer une partie intermédiaire de l'aiguille à double extrémité.

6. Ensemble de distribution médicale pré-rempli selon la revendication 1, dans lequel la base de protection (220) est retenue entre la au moins une bride de flacon (118) et le moyeu d'aiguille (150).

7. Ensemble de distribution médicale pré-rempli selon la revendication 1, dans lequel le collier (132) est d'un seul tenant avec le moyeu d'aiguille (150).

8. Ensemble de distribution médicale, comprenant :
un collier (132) présentant une première extrémité définissant une douille de montage comprenant un siège intérieur pouvant fonctionner pour accepter une bride extérieure d'un flacon (110) à joint de remplissage par soufflage (BFS) qui est apparié axialement, et une seconde extrémité définissant une douille de moyeu comprenant une caractéristique d'appariement de collier ;
un moyeu d'aiguille (150) comprenant au moins une caractéristique d'appariement de moyeu appariée en coopération avec la caractéristique d'appariement de collier, le moyeu d'aiguille étant couplé à une aiguille à double extrémité disposée à travers le moyeu d'aiguille ;
une protection d'aiguille (200) comprenant une base de protection (220) couplée au collier (132), un élément de protection (224), et un élément d'articulation (218) reliant de manière flexible la base de protection et l'élément de protection, dans lequel l'élément de protection comprend un élément de rétention présentant une barbe de rétention configurée pour capturer l'extrémité de distribution de l'aiguille à double extrémité dans un espace de rétention de l'élément de rétention, et dans lequel la base de protection comprend en outre au moins une encoche circonférentielle qui peut être couplée à au moins une bride de flacon du flacon BFS.

9. Ensemble de distribution médicale selon la revendication 8, dans lequel l'élément de protection (224) est mis en rotation autour de l'élément d'articulation et couplé à l'extrémité d'administration de l'aiguille après le retrait du capuchon.

10. Ensemble de distribution médicale selon la revendication 8, comprenant en outre un joint d'étanchéité de moyeu d'aiguille amovible recouvrant une ouverture de la douille de montage.

11. Ensemble de distribution médicale selon la revendication 8, dans lequel le collier (132) est d'un seul tenant avec le moyeu d'aiguille.
